(19) Europäisches Patentamt
European Patent Office
Office européen des brevets

(11) Veröffentlichungsnummer: **0 462 468 B1**

# (12) EUROPÄISCHE PATENTSCHRIFT

(45) Veröffentlichungstag der Patentschrift: **28.09.94**

(21) Anmeldenummer: **91109388.8**

(22) Anmeldetag: **07.06.91**

(51) Int. Cl.5: **C07C 211/41**, C07C 217/54, A61K 31/135, C07D 211/14, A61K 31/445

## (54) 9-Amino-2-phenylbicyclo[3.3.1]nonane und 9-Amino-2-phenylbicyclo[3.3.1]-non-2-ene und diese enthaltende therapeutische Mittel.

(30) Priorität: **21.06.90 DE 4019782**

(43) Veröffentlichungstag der Anmeldung: **27.12.91 Patentblatt 91/52**

(45) Bekanntmachung des Hinweises auf die Patenterteilung: **28.09.94 Patentblatt 94/39**

(84) Benannte Vertragsstaaten: **AT BE CH DE DK FR GB IT LI NL SE**

(56) Entgegenhaltungen:
**EP-A- 0 008 163**
**DE-A- 2 619 617**

**JOURNAL OF THE AMERICAN CHEMICAL SOCIETY, Band 72, Nr. 8, 15. August 1950, Seiten 3405-3410; A.C. COPE et al.: "Cyclic Polyolefins. X. Synthesis of Phenylcycloöcta-1,3-diene"**

(73) Patentinhaber: **BASF Aktiengesellschaft**
**Carl-Bosch-Strasse 38**
**D-67063 Ludwigshafen (DE)**

(72) Erfinder: **Schlecker, Rainer, Dr.**
**Suedring 8**
**W-6719 Bissersheim (DE)**
Erfinder: **Hofmann, Hans Peter, Dr.**
**Untere Hart 12**
**W-6703 Limburgerhof (DE)**
Erfinder: **Szabo, Laszlo, Dr.**
**Kastellweg 10**
**W-6900 Heidelberg (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

JOURNAL OF THE CHEMICAL SOCIETY Band 16, 1968, Seiten 2032-2039; R.A. APPLETON et al.: "Studies on Bicyclononanes. Part V. Formation ofSubstituted Bicyclononanes from Enamines"

JOURNAL OF THE AMERICAN CHEMICAL SOCIETY Band 96, Nr. 1, 9. Januar1974, Seiten 149-154; H. CALDARARU et al.: "Electron Spin Resonance Study ofSome Iminoxy Radicals. Stereochemistry of Bicyclic Systems"

## Beschreibung

Die Erfindung betrifft 9-Amino-2-phenylbicyclo[3.3.1]nonane und 9-Amino-2-phenylbicyclo[3.3.1]non-2-ene und diese enthaltende therapeutische Mittel.

Der Erfindung lag die Aufgabe zugrunde, neue Arzneimittel, vor allem zur Behandlung zentralnervöser Erkrankungen, zu entwickeln.

Es wurde nun gefunden, daß 9-Amino-2-phenylbicyclo[3.3.1]nonane und 9-Amino-2-phenylbicyclo-[3.3.1]non-2-ene der allgemeinen Formel I

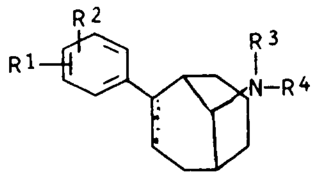

I

in der

$R^1$ und $R^2$, die gleich oder verschieden sind, ein Wasserstoff- oder Halogenatom, eine Alkyl-, Alkoxy-, Dialkylamino-, Trifluormethyl-, Hydroxy-, Alkylthio-, Alkylsulfonyl- oder Nitrogruppe,

$R^3$ und $R^4$, die gleich oder verschieden sind, Wasserstoff, eine Alkylgruppe mit 1 bis 5 oder eine Alkenyl- oder Alkinylgruppe mit jeweils 2 bis 5 Kohlenstoffatomen oder Benzyl darstellen, wobei $R^3$ und $R^4$ auch zusammen eine Kette von drei bis sieben gesättigten Kohlenstoffatomen bilden können, die durch einen Phenylring substituiert sein kann,

und wobei für eine Einfach- oder Doppelbindung steht, wertvolle pharmakologische Eigenschaften, insbesondere mit Wirkung auf das zentrale Nervensystem besitzen.

Die Verbindungen der Formel I lassen sich durch reduktive Aminierung der Ketone II herstellen.

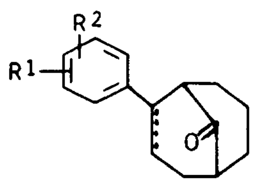

II

Hierin haben die Symbole $R^1$ und $R^2$ sowie die gleiche Bedeutung wie in Formel I. Die Verfahren zur reduktiven Aminierung von Ketonen sind allgemein bekannt und beispielsweise in Houben-Weyl, Methoden der organischen Chemie, Band 4/1d, S. 355-364 und 4/1c, S. 427-457 beschrieben. Zur Reduktion können komplexe Metallhydride wie $NaBH_4$ oder $NaBH_3CN$ oder die katalytische Hydrierung in Gegenwart eines Amins $NHR^3R^4$ eingesetzt werden. Die Ketone II werden nach literaturbekannten Verfahren hergestellt (endo-II: gesättigt und mit Doppelbindung: A.C. Cope et al., JACS, 72, 3405-3410 (1950); exo-II gesättigt und mit Doppelbindung: R.A. Appleton et al., J. Chem. Soc. (C), 1968, 2032-2039).

In der Regel handelt es sich bei den erfindungsgemäßen Verbindungen um Stereoisomeren-Mischungen. Unter den Anspruch fallen aber auch die isolierten exo- und endo-Phenylisomeren Ia und Ib sowie deren Stereoisomere.

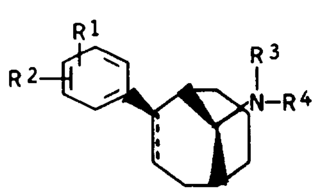

Ia

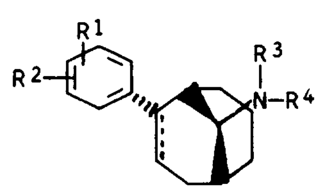

Ib

Die erfindungsgemäßen Verbindungen Ia und Ib stellen ihrerseits wiederum jeweils eine Diastereomerenmischung dar, die sich chromatographisch oder durch Umkristallisation trennen läßt. Die Auftrennung der reinen Diastereomere in die Enantiomeren gelingt nach an sich bekannten Methoden, beispielsweise durch

Überführung in diastereomere Salze optisch aktiver Hilfssäuren wie Dibenzoylweinsäure, Ditoluylweinsäure oder Campher-10-sulfonsaure und deren Trennung durch Kristallisation. Weiterhin gelingt die Trennung der Enantiomeren durch Chromatographie an chiralen Phasen.

Gegebenenfalls werden die erhaltenen erfindungsgemäßen Verbindungen in ihre Säureadditionssalze mit physiologisch verträglichen Säuren überführt. Als übliche physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere können aus Fortschritte der Arzneimittelforschung, Bd. 10, S. 224 f, Birkhäuser Verlag, Basel und Stuttgart, 1966, entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, bei Spielsweise einem niederen Alkohol, wie Methanol, Ethanol oder Propanol, einem Ether wie Methyl-t-butylether, einem Keton wie Aceton oder Methylethylketon oder einem Ester wie Essigsäureethylester erhalten. Zur besseren Kristallabscheidung können auch Mischungen der genannten Lösungsmittel verwendet werden. Darüber hinaus können pharmazeutisch vertretbare wäßrige Lösungen von Säureadditionsverbindungen der erfindungsgemäßen Verbindungen I durch Auflösen der freien Basen in einer wäßrigen Säurelösung hergestellt werden.

Die erfindungsgemäßen Verbindungen eignen sich zur Bekämpfung von Krankheiten, insbesondere zur Behandlung von Erkrankungen des zentralen Nervensystems (ZNS). Sie können in üblicher Weise oral oder parenteral (subkutan, intravenös, intramuskulär, intraperitoneal) verabfolgt werden. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 1000 mg pro Patient und Tag bei oraler Gabe und etwa 1 bis 500 mg pro Patient und Tag bei parenteraler Gabe.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sukker et al: Pharmazeutische Technologie, Thieme-Verlag, Stuttgart, 1978). Die so erhaltenen Applikationsformen enthalten den Wirkstoff normalerweise in einer Menge von 1 bis 99 Gew.%.

Beispiel 1

9-Amino-2-endo(4-methoxyphenyl)bicyclo[3.3.1]nonan

Zu 5,1 g 2-endo(4-Methoxyphenyl)bicyclo[3.3.1]nonan-9-on und 19,4 g Ammoniumacetat in 60 ml abs. Methanol wird eine Lösung von 1,0 g $NaBH_3CN$ in wenig Methanol unter Eiskühlung zugetropft. Man läßt über Nacht rühren, destilliert das Lösungsmittel ab und versetzt den Rückstand mit Eis. Die Mischung wird mit 1n HCl auf pH 1 gebracht, mit Ether extrahiert und mit 1n NaOH alkalisch gestellt. Die Wasserphase wird mit Ether extrahiert, die org. Phase abgetrennt und getrocknet. Nach Zutropfen von etherischer HCl erhält man 4,0 g 9-Amino-2-endo(4-methoxyphenyl)bicyclo[3.3.1]nonan-hydrochlorid, Fp. 232 °C.

Beispiel 2

9-Allylamino-2-endo(4-methylphenyl)bicyclo[3.3.1]nonan

Zu 4,8 g 2-endo(4-Methylphenyl)bicyclo[3.3.1]nonan-9-on, 22,1 g Allylammoniumacetat in 50 ml abs. Methanol wird eine Lösung von 1,0 g $NaBH_3CN$ in wenig Methanol zugetropft. Nach Rühren über Nacht wird wie in Beispiel 1 beschrieben aufgearbeitet. Man erhält 4,3 g 9-Allylamino-2-endo(4-methylphenyl)-biyclo[3.3.1]nonan-hydrochlorid, Fp. 202 °C.

In analoger Weise wurden, ausgehend von den entsprechenden Ketonen, unter anderem die nachstehenden erfindungsgemäßen Verbindungen hergestellt.

Tabelle

| Beisp. | $R^1$ | $R^2$ | $NR^3$, $R^4$ | exo/endo | I a) | Fp. (Hydrochlorid) |
|---|---|---|---|---|---|---|
| 3 | 4-$OCH_3$ | H | $NH_2$ | endo | I | 120-123 b) |
| 4 | H | H | $NH_2$ | endo | I | 229-231 |
| 5 | H | H | $N(CH_3)_2$ | - | II | 244-245 |
| 6 | H | H | $NHCH_3$ | - | II | 218-221 |
| 7 | H | H | $NHCH_3$ | endo | I | 255-258 |
| 8 | H | H | $NHC_2H_5$ | endo | I | 227-230 |
| 9 | H | H | 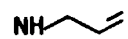 | endo | I | 189-191 |
| 10 | H | H | NH-$C_3H_7$ | endo | I | 221-224 |
| 11 | H | H | $N(CH_3)_2$ | endo | I | 231-234 |
| 12 | H | H | NH-$C_3H_7$ | - | II | 248-251 |
| 13 | H | H | 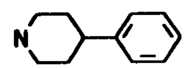 | endo | I | 277-280 |
| 14 | H | H | 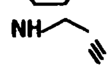 | exo | I | 167-170 |
| 15 | H | H | $NHC_4H_9$ | exo | I | 216-220 |
| 16 | H | H | 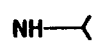 | exo | I | 223-225 |

Tabelle (Fortsetzung)

<table>
<tr><th>Beisp.</th><th>$R^1$</th><th>$R^2$</th><th>$NR^3, R^4$</th><th>exo/endo</th><th>|:a)</th><th>Fp. (Hydrochlorid)</th></tr>
<tr><td>17</td><td>4-Cl</td><td>H</td><td>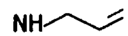</td><td>-</td><td>||</td><td>203</td></tr>
<tr><td>18</td><td>4-Cl</td><td>H</td><td>$NHCH_3$</td><td>-</td><td>||</td><td>249</td></tr>
<tr><td>19</td><td>4-Cl</td><td>H</td><td>$NH_2$</td><td>-</td><td>||</td><td>247</td></tr>
<tr><td>20</td><td>4-F</td><td>H</td><td>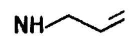</td><td>-</td><td>||</td><td>216-218</td></tr>
<tr><td>21</td><td>4-F</td><td>H</td><td>$NHCH_3$</td><td>-</td><td>||</td><td>224</td></tr>
<tr><td>22</td><td>4-F</td><td>H</td><td>$NH_2$</td><td>-</td><td>||</td><td>238-239</td></tr>
<tr><td>23</td><td>$4-CH_3$</td><td>H</td><td>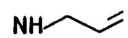</td><td>-</td><td>||</td><td>213-215</td></tr>
<tr><td>24</td><td>$4-CH_3$</td><td>H</td><td>$NHCH_3$</td><td>-</td><td>||</td><td>240</td></tr>
<tr><td>25</td><td>$4-CH_3$</td><td>H</td><td>$NH_2$</td><td>-</td><td>||</td><td>239</td></tr>
<tr><td>26</td><td>$4-OCH_3$</td><td>H</td><td>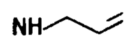</td><td>-</td><td>||</td><td>219</td></tr>
<tr><td>27</td><td>$4-OCH_3$</td><td>H</td><td>$NHCH_3$</td><td>-</td><td>||</td><td>198</td></tr>
<tr><td>28</td><td>$4-OCH_3$</td><td>H</td><td>$NH_2$</td><td>-</td><td>||</td><td>223-224</td></tr>
<tr><td>29</td><td>H</td><td>H</td><td>$NH_2$</td><td>-</td><td>||</td><td>258</td></tr>
<tr><td>30</td><td>4-F</td><td>H</td><td>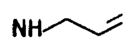</td><td>endo</td><td>|</td><td>222</td></tr>
<tr><td>31</td><td>4-F</td><td>H</td><td>$NHCH_3$</td><td>endo</td><td>|</td><td>283</td></tr>
</table>

Tabelle (Fortsetzung)

| Beisp. | $R^1$ | $R^2$ | $NR^3, R^4$ | exo/endo | |:a) | Fp. (Hydrochlorid) |
|---|---|---|---|---|---|---|
| 32 | 4-$(CH_3)_3C$- | H | NH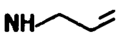 | - | II | 215 |
| 33 | 4-$(CH_3)_3C$- | H | $NH_2$ | - | II | 273 |
| 34 | 4-$(CH_3)_3C$- | H | $NHCH_3$ | - | II | 309 |
| 35 | 4-$(CH_3)_3C$- | H | NH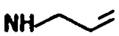 | endo | I | 227 |
| 36 | 4-$(CH_3)_3$- | H | $NH_2$ | endo | I | 256 |
| 37 | 4-$(CH_3)_3C$- | H | $NHCH_3$ | endo | I | 351 |
| 38 | 4-Cl | 3-Cl | NH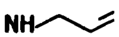 | - | II | 215 |
| 39 | 4-Cl | 3-Cl | $NH_2$ | - | II | 236 |
| 40 | 4-Cl | 3-Cl | $NHCH_3$ | - | II | 261 |
| 41 | 4-$OCH_3$ | H | NH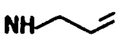 | endo | I | 156 |
| 42 | 4-$OCH_3$ | H | $NHCH_3$ | endo | I | 263 |
| 43 | 4-$CH_3$ | H | NH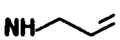 | endo | I | 202 |
| 44 | 4-$CH_3$ | H | $NHCH_3$ | endo | I | 261-263 |
| 45 | 4-$CH_3$ | H | $NH_2$ | endo | I | 251 |
| 46 | 4-$OCH_3$ | H | $NH_2$ | endo | I | 232 |

Tabelle (Fortsetzung)

| Beisp. | R1 | R2 | NR3, R4 | exo/endo | ⁝a) | Fp. (Hydrochlorid) |
|---|---|---|---|---|---|---|
| 47 | 4-Cl | 3-Cl | $NHCH_3$ | endo | \| | 277 |
| 48 | 4-Cl | 3-Cl | NH 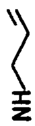 | endo | \| | 171 |
| 49 | 4-Cl | 3-Cl | $NH_2$ | endo | \| | 256 |
| 50 | 4-Cl | H | $NH_2$ | endo | \| | 249 |
| 51 | 4-Cl | H | NH 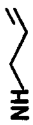 | endo | \| | 200 |
| 52 | 4-Cl | H | $NH_2$ | endo | \| | 262 |
| 53 | 4-Cl | H | $NHCH_3$ | endo | \| | 292 |

a) || = Doppelbindung,

| = Einfachbindung

b) freie Base

Beispiel 54

Diastereomerentrennung von 9-Amino-2-endo-phenylbicyclo[3.3.1]nonan

50 g Produkt aus Beispiel 4 werden dreimal aus Wasser umkristallisiert. Man erhalt 15 g 9-syn-Amino-2-endo-phenylbicyclo[3.3.1]nonan, Fp. 210-212 °C.

Die wäßrigen Mutterlaugen werden vereinigt, mit 1n NaOH alkalisch gestellt und mit etherischer HCl versetzt. Das Kristallisat wird abgesaugt und dreimal aus Isopropanol umkristallisiert. Man erhält 3 g 9-anti-Amino-2-endo-phenylbicyclo[3.3.1]nonan, Fp. 278 °C. Die Zuordnung der relativen Konfiguration erfolgte durch die $^{13}$C-NMR-Spektren der Substanzen.

## Patentansprüche

1. 9-Amino-2-phenylbicyclo[3.3.1]nonane und 9-Amino-2-phenylbicyclo[3.3.1]non-2-ene der allgemeinen Formel I

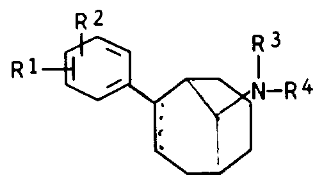

I,

in der
$R^1$ und $R^2$, die gleich oder verschieden sind, ein Wasserstoff- oder Halogenatom, eine Alkyl-, Alkoxy-, Dialkylamino-, Trifluormethyl-, Hydroxy-, Alkylthio-, Alkylsulfonyl- oder Nitrogruppe,
$R^3$ und $R^4$, die gleich oder verschieden sind, Wasserstoff, eine Alkylgruppe mit 1 bis 5 oder eine Alkenyl- oder Alkinylgruppe mit jeweils 2 bis 5 Kohlenstoffatomen oder Benzyl darstellen, wobei $R^3$ und $R^4$ auch zusammen eine Kette von drei bis sieben gesättigten Kohlenstoffatomen bilden können, die durch einen Phenylring substituiert sein kann,
und wobei ⁞ für eine Einfach- oder Doppelbindung steht.

2. 9-Amino-2-phenylbicyclo[3.3.1]nonane und 9-Amino-2-phenylbicyclo[3.3.1]non-2-ene der allgemeinen Formel I nach Anspruch 1, dadurch gekennzeichnet, daß $R^1$ und $R^2$ unabhängig voneinander Wasserstoff, Fluor, Chlor, $C_1$-$C_4$-Alkyl oder Methoxy,
$R^3$ und $R^4$ unabhängig voneinander Wasserstoff, $C_1$-$C_4$-Alkyl, Allyl, Propargyl bedeuten, oder daß $R^3$ und $R^4$ gemeinsam eine gesättigte Kette von 4 oder 5 Kohlenstoffatomen bilden, die durch einen Phenylring substituiert sein kann.

3. Arzneimittel zur peroralen oder parenteralen Anwendung, das neben üblichen galenischen Hilfsstoffen eine Verbindung der Formel I nach Anspruch 1 oder 2 als Wirkstoff enthält.

4. Verwendung einer Verbindung der Formel I nach Anspruch 1 oder 2 zur Herstellung eines Arzneimittels.

## Claims

1. A 9-amino-2-phenylbicyclo[3.3.1]nonane or 9-amino-2-phenylbicyclo[3.3.1]non-2-ene of the formula I

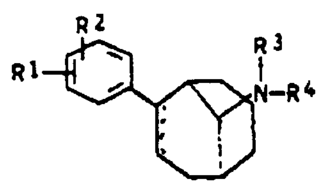

I,

where
$R^1$ and $R^2$, which are identical or different, are a hydrogen or halogen atom, an alkyl, alkoxy, dialkylamino, trifluoromethyl, hydroxyl, alkylthio, alkylsulfonyl or nitro group,
$R^3$ and $R^4$, which are identical or different, are hydrogen, an alkyl group with 1 to 5 or an alkenyl or alkynyl group with in each case 2 to 5 carbon atoms or benzyl, it also being possible for $R^3$ and $R^4$ together to form a chain which has from three to seven saturated carbon atoms and can be substituted by a phenyl ring,
and where ⁞ is a single or double bond.

2. A 9-amino-2-phenylbicyclo[3.3.1]nonane or 9-amino-2-phenylbicyclo[3.3.1]non-2-ene of the formula I as claimed in claim 1, wherein $R^1$ and $R^2$ are, independently of one another, hydrogen, fluorine, chlorine,

$C_1$-$C_4$-alkyl or methoxy,

$R^3$ and $R^4$ are, independently of one another, hydrogen, $C_1$-$C_4$-alkyl, allyl, propargyl, or wherein $R^3$ and $R^4$ together form a saturated chain which has 4 or 5 carbon atoms and can be substituted by a phenyl ring.

**3.** A drug for oral or parenteral use which, besides conventional pharmaceutical ancillary substances, contains a compound of the formula I as claimed in claim 1 or 2 as active substance.

**4.** The use of a compound of the formula I as claimed in claim 1 or 2 for producing a drug.

## Revendications

**1.** 9-Amino-2-phénylbicyclo[3.3.1]nonanes et 9-amino-2-phénylbicyclo[3.3.1]non-2-ènes de la formule générale I

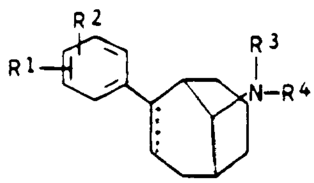

I,

dans laquelle

$R^1$ et $R^2$, qui sont identiques ou différents, représentent chacun un atome d'hydrogène ou un atome d'halogène, un radical alkyle, alcoxy, dialkylamino, trifluorométhyle, hydroxyle, alkylthio, alkylsulfonyle ou nitro,

$R^3$ et $R^4$, qui sont identiques ou différents, représentent un atome d'hydrogène, un radical alkyle qui comporte de 1 à 5 atomes de carbone, ou un radical alcényle ou alcynyle qui comportent chacun 2 à 5 atomes de carbone, ou le radical benzyle, où $R^3$ et $R^4$ peuvent aussi former ensemble une chaîne de 3 à 7 atomes de carbone saturés, qui peut être substituée par un noyau phényle

et dans laquelle ⁝ représente une simple liaison ou une double liaison.

**2.** 9-Amino-2-phénylbicyclo[3.3.1]nonanes et 9-amino-2-phénylbicyclo[3.3.1]non-2-ènes de la formule générale I suivant la revendication 1, caractérisés en ce que $R^1$ et $R^2$ représentent, indépendamment l'un de l'autre, chacun un atome d'hydrogène, de fluor, de chlore, un radical alkyle en $C_1$-$C_4$ ou le radical méthoxy,

$R^3$ et $R^4$, indépendamment l'un de l'autre, représentent chacun un atome d'hydrogène, un radical alkyle en $C_1$-$C_4$,allyle, propargyle, ou en ce que $R^3$ et $R^4$ forment ensemble une chaîne saturée de 4 ou de 5 atomes de carbone, qui peut être substituée par un noyau phényle.

**3.** Médicaments destinés à l'administration perorale ou parentérale, qui contient un composé de la formule I selon la revendication 1 ou 2 en plus d'adjuvants galéniques habituels.

**4.** Utilisation d'un composé de la formule I suivant la revendication 1 ou 2 pour la préparation d'un médicament.